# EUROPEAN PATENT APPLICATION

(11) **EP 3 437 496 A1**
(43) Date of publication of application: **06.02.2019**
(21) Application number: 17184550.6
(22) Date of filing: 02.08.2017
(51) Int. Cl.: A24F 47/00, A61M 11/04, A61M 15/06

(54) **RAPID REPLACE HYGIENIC COIL SYSTEM**

(71) Applicant: BSMW Limited, Linthwaite Huddersfield HD7 5QH (GB)
(72) Inventor: Wilson, Ben, Marple Bridge, Stockport SK6 5NX (GB); Zee, Kum Wing, Sothall, Sheffield S20 2QQ (GB); France, Simon, Bramley, Rotherdam S66 3XA (GB)
(74) Representative: Kernebeck, Thomas

(57) **Abstract**

An integrally formed coil member (1) is disclosed, which includes a mouthpiece (2), a vaping chamber (3), and a heating coil (16). When the heating coil (16) has deteriorated and needs to be replaced, the entire coil member (1) can be exchanged rapidly and subsequently disposed. The coil member (1) may also be left in a vaping tank (11) when the vaping device (10) is not in use.

## Description

The invention relates to a vaping device and a coil member for a vaping device, and more particularly to disposable coils for electronic cigarettes or vaping devices.

The dangers of smoking are well documented. Electronic cigarettes, or vaping devices, avoid the generation of smoke from burning tobacco products by evaporating an "electronic liquid" or e-liquid. Specifically, the e-liquid is vaporized in the vaping device and the resulting vapour is consumed instead of the smoke that is created when burning tobacco products, e.g., cigarettes.

Heating coils are used in the electronic cigarettes or vaping devices to heat up and vaporize the e-liquid. The heating coils heat up the e-liquid when connected to a power source, typically a battery. Further, heating coils must be replaced regularly as they deteriorate over time. However, the way in which coils are replaced in known devices is not simple, which causes frustration for consumers and may revert them back to simpler, cartridge type products or even cigarettes. For example, when replacing coils in products currently on the market, the consumers' hands may come into contact with the e-liquid stored in the tank, which may in some cases cause skin irritations. Moreover, the replacement may be cumbersome and result in accidental change of user settings.

It is therefore an object of the invention to provide a simpler, faster, and cleaner way to replace a heating coil of a vaping device than that of the known products on the market, making coil replacement more convenient and hassle-free for the consumer.

This object is achieved by a coil member having the features according to claim 1 and by a vaping device having the features according to claim 8. Further preferred embodiments are provided in the dependent claims.

A coil member or a vaping device according to the invention includes a heating coil arranged at an inlet end of a vaping chamber, and a mouthpiece arranged at an outlet end of the vaping chamber, characterized in that the vaping chamber and the mouthpiece are integrally formed.

Thus, the inventive coil is provided as a coil member that is integrally formed by a vaping chamber and a mouth piece and when it is time to replace the heating coil, the entire coil member is removed by unscrewing the mouthpiece from the vaping tank to which the coil member is connected, thereby taking the heating coil with it. The integrally formed mouthpiece, vaping chamber, and coil are then disposed in a hygienic manner. Mouthpiece and coil are disposed as one piece which meets the requirements of the European Union's Tobacco Products Directive which took effect on May 20, 2017.

In the related art, the coil is a stand-alone coil and so is the mouthpiece. By contrast, according to the invention, the mouth piece is connected to the coil and the coil is replaced by unscrewing the mouth piece together with the coil. This prevents the coil from coming in contact with the consumers hands and hence with the e-liquid.

According to a preferred embodiment of the invention, the coil member is provided with a vaping chamber that has an exterior annular groove. According to another preferred embodiment of the invention, a seal is provided in the exterior annular groove. Preferably, the seal is a silicone ring or a vulcanized natural rubber ring. The silicon ring or vulcanized natural rubber ring incorporated below the base of the mouthpiece ensures that no liquid is lost.

According to a further preferred embodiment of the invention, the coil member has an inflow opening that is provided adjacent to the inlet end of the vaping chamber. According to another preferred embodiment of the invention, a flange is provided adjacent to the inflow opening. In yet another preferred embodiment of the invention, a first fastening unit is disposed on the coil member.

A vaping device according to the invention includes a vaping tank for holding an e-liquid and a coil member insertable into a receiving bore of the vaping tank, wherein the coil member includes a heating coil arranged at an inlet end of a vaping chamber, and a mouthpiece arranged at an outlet end of the vaping chamber, and wherein the vaping chamber and the mouthpiece are integrally formed.

According to a preferred embodiment of the invention, the vaping device includes a floating internal collar which is arranged in the receiving bore. Preferably, the floating internal collar seals an outlet opening in the receiving bore when the coil member is not inserted into the receiving bore.

This prevents the e-liquid from entering the vaping chamber when the coil is not in situ.

Preferably, the floating internal collar is pushed away from the outlet opening when the coil member is inserted into the receiving bore. When the coil member is inserted, the collar is pushed down by a flange on the coil to allow e-liquid to flow into the vaping chamber and to soak into the coils wadding. The flange may have a dimension of 0.5 mm.

In an advantageous embodiment, a first fastening unit is provided on the coil member and a second fastening unit, which cooperates with the first fastening unit, is provided on the vaping tank.

According to another preferred embodiment of the invention, the first fastening unit and the second fastening unit cooperate to hold the vaping device in an operating state in which the e-liquid flows from the outlet opening of the vaping tank through the inflow opening of the coil member. Preferably, the first fastening unit and the second fastening unit cooperate to hold the vaping device in a storage state in which the outlet opening on the vaping tank is sealed by an external surface of the coil member. According to a particularly preferred embodiment of the invention, the first fastening unit is an external thread and the second fastening unit is an internal thread.

Further features of the invention emerge from the claims, the figures and the description of the figures. The features and feature combinations referred to in the above description and the features and combinations of features in the description of the figures and/or only shown in the figures may be also be used in other combinations or on their own without departing from the scope of the invention. Embodiments of the invention that are not explicitly shown and described in the figures but which emerge from the described embodiments by way of separate feature combinations should also be considered to be disclosed. Therefore, embodiments and feature combinations which do not have all of the features of an originally formulated independent claim should also be considered to be disclosed.

Below, advantageous exemplary embodiments of the invention, which are schematically depicted in the drawings, are described, wherein:
Fig. 1 shows a prior art mouthpiece 2' and housing 3';
Fig. 2 shows the inventive integrally formed coil member 1;
Fig. 3 shows coil member 1 that can be inserted into vaping tank 11;
Fig. 4 shows a top view of a vaping device in which the coil member can be switched between a use position 31 and a storage position 32;
Fig. 5a shows a side view of the coil member inserted into the vaping tank in a use position 31;
Fig. 5b shows a side view of the coil member inserted into the vaping tank in a storage position 32; and
Fig. 6 shows in detail flange 8 provided on the coil member 1.

The terms electronic cigarette, e-cigarette, and vaping device are often used interchangeably. Nonetheless, the term electronic cigarette is sometimes used to indicate that the e-liquid to be vaporized contains nicotine, whereas a vaping device is understood to mean a device in which an e-liquid without nicotine is used. The inventive heating coil and vaping device work equally well in either case. Thus, the term vaping device is used throughout this disclosure regardless of the type of e-liquid used and encompasses vaping devices as well as electronic cigarettes or e-cigarettes.

Additionally, the term vaping tank is defined herein to encompass the housing in which a tank portion for holding the e-liquid is provided. However, the vaping tank may house other components in addition to the tank portion, such as a battery for powering the heating coil, a memory to store user settings, or a display for displaying the fill level of the e-liquid in the tank portion. Thus, the term vaping tank herein means any housing that has at least a tank portion for holding an e-liquid.

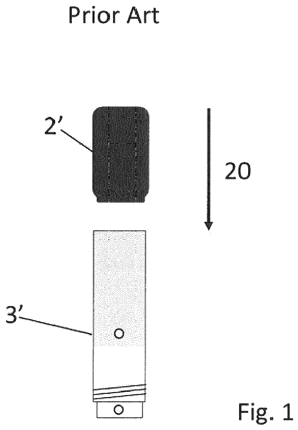

In the prior art as shown in Fig. 1, mouthpiece 2' and housing 3', which houses a vaping chamber and a heating coil, are separate parts. Specifically, mouthpiece 2' is inserted into housing 3' as indicated by arrow 20. When the deteriorated heating coil needs to be replaced, the mouthpiece is detached first before the housing 3' with the heating coil can be replaced. Sometimes, the housing 3', which includes the heating coil is referred to as "the coil" and replacing the coil means that the entire housing 3' is replaced. Likewise, mouthpiece 2' is replaceable separately from housing 3'.

For the purposes of clarity, the inventive housing unit that includes the heating coil is herein referred to as the coil member to indicate that a coil replacement involves replacing of the entire coil member, and not merely the resistive wire forming the heating coil.

When a heating coil in a prior art vaping device needs to be replaced, the vaping chamber typically still contains e-liquid and/or condensate. Because vaping chamber and mouthpiece are in fluid communication, e-liquid may spill through the opening on the housing 3' for receiving mouthpiece 2'. In some instances, it also becomes difficult to remove the housing 3' from the vaping tank or the mouthpiece 2' may come off unexpectedly during the replacement process.

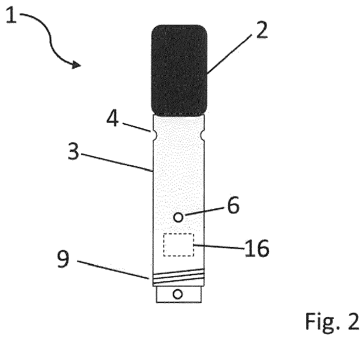

To avoid the disadvantages of the prior art, and to provide an easy, clean, and efficient way to replace a heating coil, the instant coil member was invented. In the exemplary embodiment of the invention shown in Fig. 2, an integrally formed coil member 1 includes a vaping chamber 3 and a mouthpiece 2, which are in fluid communication. The coil member 1 has an elongated shape, typically cylindrical, and houses a heating coil 16 in the vicinity of inflow opening 6. Commonly, a wicking material (not shown) is provided with the heating coil 16. The wicking material, sometimes also referred to as wadding, initially absorbs the e-liquid before the e-liquid evaporates when the heating coil 16 is powered up. Various arrangements of the wicking material and the heating coil 16 are possible. However, the various arrangements are not an objective of this disclosure. Accordingly, this disclosure is not limited to the arrangement shown in Fig. 2, but encompasses other arrangements known to the skilled artisan.

The vaping chamber has an inlet end 7 and an outlet end 7'. The resistive wire of the heating coil 16 heats up when an electric current, typically provided by a battery, flows through the heating coil 16 which vaporizes the e-liquid in the wicking material. Air and vaporized e-liquid form a vapour that flows in the general direction of a longitudinal axis of the coil member from the inlet end 7 to the outlet end 7' of the vaping chamber 3 into the mouthpiece 2. An opening 30 in the mouthpiece allows the user to consume the vapour. In proximity to the mouthpiece, i.e., toward the outlet end of the vaping chamber, an exterior annular groove 4 is formed in the external wall of the vaping chamber. Toward the inlet end 7, a first fastening unit 9 is arranged on the coil member 1.

As noted above, Fig. 2 shows coil member 1 that includes a vaping chamber 3, which has an exterior annular groove 4. Typically, the annular groove is provided toward the outlet end 7' of the vaping chamber 3. The exact location of annular groove 4 is not critical, provided that the annular groove is closer to the outlet end 7' than inlet opening 6.

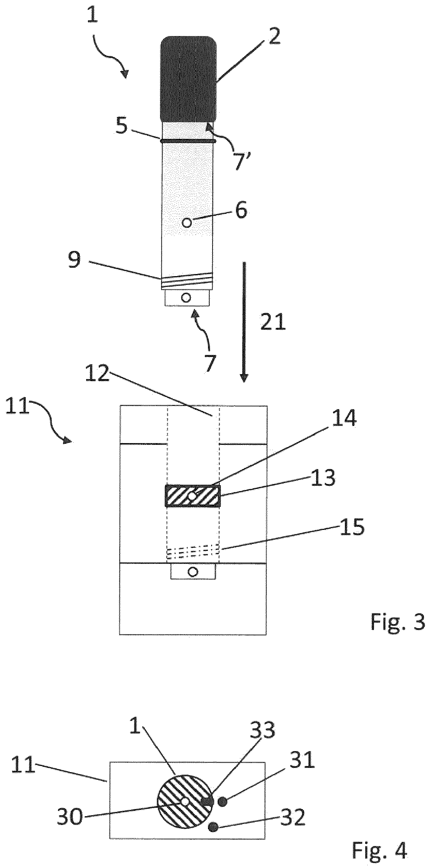

Fig. 3 shows coil member 1 that is inserted into receiving bore 12 of vaping tank 11 by pushing the coil member in the direction of arrow 21. A floating internal collar 13 is arranged within the receiving bore 12. The floating internal collar 13 seals an outlet opening 14 arranged within the receiving bore 12 when the coil member 1 is not inserted in the vaping tank 11. The outlet opening 14 is in fluid communication with the tank portion of vaping tank 11, where the tank portion holds the e-liquid. When the coil member 1 is inserted in the vaping tank 11, flange 8 catches the floating internal collar 13 and pushes it down, such that the outlet opening 14 becomes exposed. When the coil member is fully inserted and the first fastening unit 9 and the second fastening unit 15 are engaged, the coil member 1 can be held in an operating position 31, in which the outlet opening 14 and the inlet opening 6 are aligned such that the e-liquid can flow from the vaping tank 11 into the vaping chamber 3.

The first fastening unit 9 may be provided as an external thread on the inlet end of the vaping chamber and the second fastening unit as a matching internal thread within the receiving bore 12. Screwing the coil member 1 into the receiving bore 12 fastens the coil member 1 to the vaping tank 11 in the use position 31 shown in Fig. 4. In a particular embodiment, partial unscrewing of the coil member results in a misalignment of inlet opening 6 and outlet opening 14, such that the vaping device may be stored without e-liquid loss. In another particular embodiment, the second fastening unit 15 is rotatably mounted in the receiving bore 12. After the coil member 1 is fully fastened, an additional application of torque rotates the mount of the second fastening unit to misalign inlet opening 6 and outlet opening 14 and to thereby achieve the storage position 32 shown in Fig. 4. Thus, the integrally formed coil member 1 also has the advantage of making the vaping device 10 more user friendly because it is simple and straightforward to switch between use and storage position by simply twisting the mouth piece 2.

Fig. 3 further shows a seal 5 that is arranged in the external annular groove 4. Typically, seal 5 is a silicone ring. However, seal 5 may also be made from other materials, such as vulcanized natural rubber. In addition, any other material known to the skilled artisan having sealing properties may also be used for the seal 5. Specifically, seal 5 prevents e-liquid, vapour, or condensate from escaping the vaping device from the gap between the receiving bore 12 and the coil member 1.

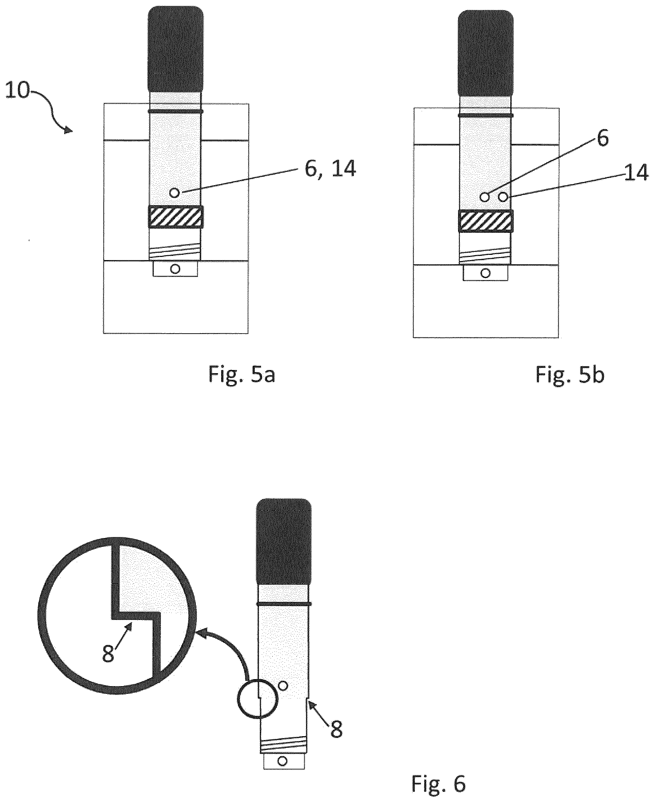

Fig. 4 shows a top view of vaping device 11 in which the coil member 1 can be switched between a use position 31 and a storage position 32. In particular, first fastening unit 9 and second fastening unit 15 can be fully engaged such that the coil member 1 is held in place when the vaping device 10 is used. Moreover, a further turn of the mouthpiece 2 results in the coil member 1 reaching storage position 32 in which the vaping tank 11 and the vaping chamber 3 are no longer in fluid communication, as illustrated in Fig. 5b. Instead, an exterior wall of the coil member 1 seals outlet opening 14 and prevents e-liquid from leaving the vaping tank 11. Accordingly, not only coil replacement, but also ordinary use of the vaping device can be performed more easily and in a more hygienic manner.

In an exemplary embodiment, a marking 33 is provided on the mouthpiece 2 to indicate if the vaping device 10 is in use position 31 or in storage position 32. Thus, the marking 33 serves as a visual indicator of the operating state of the vaping device 10.

Fig. 5a shows a side view of the coil member 1 inserted into the vaping tank 11. Specifically, Fig. 5a shows the vaping device 10 in the use position 31 in which outlet opening 14 and inlet opening 6 are aligned such that vaping tank 11 and vaping chamber 3 are in fluid communication. Seal 5 seals the coil member 1 in the receiving bore 12 and prevents vapour or e-liquid from escaping. Thus, vapour only leaves the vaping device through the mouthpiece 2.

In Fig. 5b, which is also a side view of the coil member 1 inserted into the vaping tank 11, the vaping device is in storage position 32 because inlet opening 6 and outlet opening 14 are not aligned to allow for fluid communication.

Fig. 6 shows in detail flange 8 provided on the coil member 1. The flange 8 catches floating internal collar 13 when the coil member 1 is inserted into the receiving bore 12 of vaping tank 11.

According to one aspect of the invention, a simpler, faster, and cleaner way to replace a deteriorated heating coil 16 is provided, making replacement more convenient and hassle free for the consumer. In particular, a deteriorated heating coil 16 can be replaced more easily by removing the coil member 1 from the vaping tank 11 by simply twisting the mouth piece 2.

The foregoing description of the exemplary embodiments of the disclosure illustrates and describes the present invention. Additionally, the disclosure shows and describes only the exemplary embodiments but, as mentioned above, it is to be understood that the disclosure is capable of use in various other combinations, modifications, and environments and is capable of changes or modifications within the scope of the concept as expressed herein, commensurate with the above teachings and/or the skill or knowledge of the related art.

The term "comprising" (and its grammatical variations) as used herein is used in the inclusive sense of "having" or '"including" and not in the exclusive sense of "consisting only of." The terms "a" and "the" as used herein are understood to encompass the plural as well as the singular.

### Reference list

- 1: coil member
- 2: mouthpiece
- 2': mouthpiece
- 3: vaping chamber
- 3': housing
- 4: exterior annular groove
- 5: seal
- 6: inlet opening
- 7: inlet end
- 7': outlet end
- 8: flange
- 9: first fastening unit
- 10: vaping device
- 11: vaping tank
- 12: receiving bore
- 13: floating internal collar
- 14: outlet opening
- 15: second fastening unit
- 16: heating coil
- 20: arrow
- 21: arrow
- 30: opening
- 31: use position
- 32: storage position
- 33: marking

## Claims

1. A coil member (1) for a vaping device (10) including:
- a heating coil (16) arranged at an inlet end (7) of a vaping chamber (3), and
- a mouthpiece (2) arranged at an outlet end (7') of the vaping chamber (3), **characterized in that** the vaping chamber (3) and the mouthpiece (2) are integrally formed.

2. The coil member according to claim 1, **characterized in that** the vaping chamber (3) has an exterior annular groove (4).

3. The coil member according to claim 2, **characterized in that** a seal (5) is provided in the exterior annular groove (4).

4. The coil member according to claim 3, **characterized in that** the seal (5) is a silicone ring or a vulcanized natural rubber ring.

5. The coil member according to any one of claims 1 to 4, **characterized in that** an inflow opening (6) is provided adjacent to the inlet end (7) of the vaping chamber (3).

6. The coil member according to claim 5, **characterized in that** a flange (8) is provided adjacent to the inflow opening (6).

7. The coil member according to any one of claims 1 to 6, **characterized in that** a first fastening unit (9) is disposed on the coil member (1).

8. A vaping device (10) including:
- a vaping tank (11) for holding an e-liquid, and
- a coil member (1) insertable into a receiving bore (12) of
the vaping tank (11), the coil member (1) including:
a heating coil (16) arranged at an inlet end (7) of a vaping chamber, and
a mouthpiece (2) arranged at an outlet end (7') of the vaping chamber, **characterized in that** the vaping chamber (3) and the mouthpiece (2) are integrally formed.

9. The vaping device according to claim 8, **characterized in that** a floating internal collar (13) is arranged in the receiving bore (12).

10. The vaping device according to claim 9, **characterized in that** the floating internal collar (13) seals an outlet opening (14) in the receiving bore when the coil member (1) is not inserted into the receiving bore (12).

11. The vaping device according to claim 9 or 10,
**characterized in that** the floating internal collar (13) is pushed away from the outlet opening (14) when the coil member (1) is inserted into the receiving bore (12).

12. The vaping device according to any one of claims 8 to 11,
**characterized in that** a first fastening unit (9) is provided on the coil member (1) and a second fastening unit (15), which cooperates with the first fastening unit (9), is provided on the vaping tank (11).

13. The vaping device according to claim 12, **characterized in that** the first fastening unit (9) and the second fastening unit (15) cooperate to hold the vaping device (10) in an operating state (31) in which the e-liquid flows from the outlet opening (14) of the vaping tank through the inflow opening (6) of the coil member (1).

14. The vaping device according to claim 12 or 13,
**characterized in that** the first fastening unit (9) and the second fastening unit (15) cooperate to hold the vaping device in a storage state (32) in which the outlet opening (14) on the vaping tank (11) is sealed by an external surface of the coil member (1).

15. The vaping device according to claim 12, 13 or 14,
**characterized in that** the first fastening unit (9) is an external thread and the second fastening unit (15) is an internal thread.
